(19) Europäisches Patentamt
European Patent Office
Office européen des brevets

(11) Veröffentlichungsnummer: **0 049 848**
**B1**

(12) # EUROPÄISCHE PATENTSCHRIFT

(45) Veröffentlichungstag der Patentschrift: **12.12.84**

(51) Int. Cl.³: **C 07 C 69/88**, C 07 C 67/317

(21) Anmeldenummer: **81107961.5**

(22) Anmeldetag: **06.10.81**

(54) **Verfahren zur Herstellung von 2,6-disubstituierten 4-Hydroxybenzoesäuren und deren Estern.**

(30) Priorität: **09.10.80 DE 3038131**

(43) Veröffentlichungstag der Anmeldung: **21.04.82 Patentblatt 82/16**

(45) Bekanntmachung des Hinweises auf die Patenterteilung: **12.12.84 Patentblatt 84/50**

(84) Benannte Vertragsstaaten: **CH DE FR GB LI**

(56) Entgegenhaltungen:
**US - A - 3 729 519**

**HOUBEN-WEYL Methoden der organischen Chemie, Bd. VI/1c Tl. 2 (1976), S. 670-674**

(73) Patentinhaber: **HOECHST AKTIENGESELLSCHAFT, Postfach 80 03 20, D-6230 Frankfurt am Main 80 (DE)**

(72) Erfinder: **Müller, Werner Heinrich, Dr., Taunusblick 5, D-6239 Eppstein/Taunus (DE)**
Erfinder: **Berthold, Rüdiger, Dr., Geierfeld 55, D-6232 Bad Soden am Taunus (DE)**

**EP 0 049 848 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist ein Verfahren zur Herstellung von 2,6-disubstituierten 4-Hydroxybenzoesäuren und deren Estern.

Sowohl die Ester als auch die Säuren haben eine fungicide Wirkung, ähnlich wie die wegen dieser Wirkung bekannten unsubstituierten 4-Hydroxybenzoesäureester, insbesondere der 4-Hydroxybenzoesäurebenzylester (PHB-Ester).

Das Verfahren zur Herstellung von 2,6-disubstituierten 4-Hydroxybenzoesäuren bzw. deren Estern der allgemeinen Formel

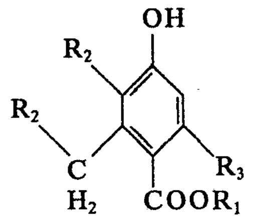

(I)

wobei die Reste $R_1$ und $R_2$ Wasserstoff oder $C_1$—$C_6$-Alkylgruppen, sowie $R_3$ eine $C_1$—$C_6$-Alkylgruppe oder ein Aromat oder Heteroaromat sein können, ist dadurch gekennzeichnet, daß man Cyclohexenoncarbonsäuren bzw. deren Ester der allgemeinen Formel

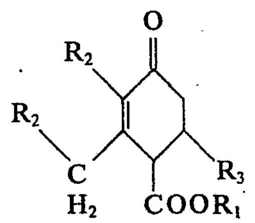

(II)

wobei die Reste $R_1$, $R_2$ und $R_3$ die oben genannte Bedeutung haben, in der Flüssigphase bei Temperaturen zwischen 140 und 350°C in Gegenwart eines Wasserstoffakzeptors an einem Katalysator dehydriert, der ein oder mehrere Edelmetalle der 8. Nebengruppe des Periodensystems enthält.

Die Ausgangsverbindungen der Formel II können auf bekannte Weise (DE-OS 2 654 850; Ann. 281, 104 (1894)) hergestellt werden:

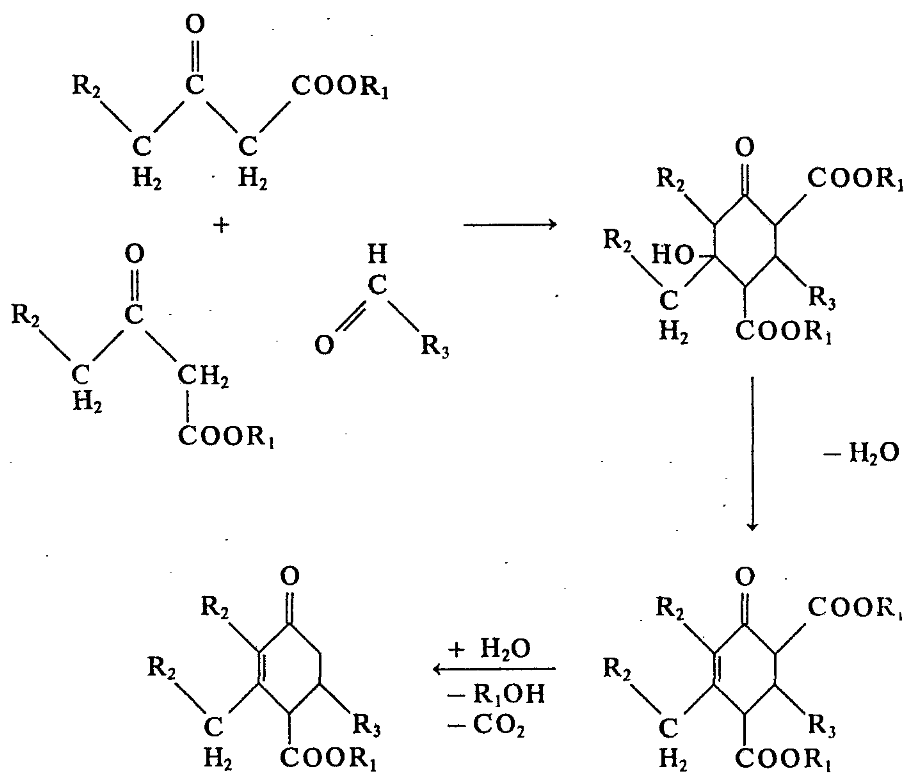

Im allgemeinen haben die drei Reste $R_2$ und $R_3$ zusammen nicht mehr als 12 C-Atome. Die für $R_1$, $R_2$ und $R_3$ in Frage kommenden $C_1$—$C_6$-Alkylreste können geradkettig, verzweigt oder cyclisch sein.

Als aromatische Reste kommen für $R_3$ vor allem Arylgruppen mit 6—14 C-Atomen in Frage, vorzugsweise der Phenyl- und der Naphthyl-Rest. Die Arylgruppen können auch substituiert sein, beispielsweise durch Halogen, insbesondere Fluor und Chlor, Alkylgruppen mit bis zu 6 C-Atomen, oder durch Trifluormethyl- oder Nitrogruppen. Aber auch Alkoxygruppen mit bis zu 6 C-Atomen, wie die Methoxy- oder Ethoxygruppe kommen in Frage. Vorzugsweise sind die Arylgruppen unsubstituiert oder nur einfach substituiert. Neben aromatischen Resten kommen für $R_3$ auch heteroaromatische Reste, beispielsweise die von Pyridin, Thiophen, Thiazol, Furan, Benzthiazol und Benzofuran abgeleiteten Reste in Frage.

Die erfindungsgemäße Dehydrierung der Cycloalkenoncarbonsäuren bzw. deren Ester der Formel II erfolgt durch Erhitzen in Gegenwart eines Dehydrierungskatalysators.

Als Dehydrierungskatalysator eignen sich beispielsweise Ruthenium, Rhodium, Palladium, Iridium und Platin oder Mischungen dieser Elemente, bevorzugt sind Palladium und Platin.

Vorzugsweise werden diese Katalysatoren auf Trägern verwendet, wie z. B. auf Kohle, Aluminiumoxid, Kieselsäure, Alumosilikaten, Spinellen, Chromia-Alumina, Zirkonoxid, Magnesiumoxid, Calciumoxid, Titanoxid und Asbest oder einer Mischung von zwei oder mehreren der genannten Träger. Besonders bewährt hat sich Palladium auf Kohle. Die Konzentration des Katalysators beträgt zweckmäßigerweise 0,02 bis 20 Gewichtsprozent, bezogen auf den Träger, vorzugsweise 0,1 bis 10 Gewichtsprozent.

Das Verfahren kann in der Flüssigphase diskontinuierlich oder kontinuierlich ausgeführt werden.

Es werden Temperaturen von 140 bis 350°C angewandt. Bevorzugt sind Temperaturen von 180 bis 260°C, da bei diesen Temperaturen eine besonders hohe Selektivität bei sehr raschem Ablauf der Dehydrierung erreicht wird.

Der Reaktionsdruck beträgt im allgemeinen 5 mbar bis 20 bar, in jedem Falle wird er aber so gewählt, daß er bei der gewählten Reaktionstemperatur zur Aufrechterhaltung einer flüssigen Phase ausreicht.

Das erfindungsgemäße Verfahren kann in Gegenwart eines Lösungsmittels ausgeführt werden. Dazu eignen sich z. B. 1) aliphatische Ether, 2) aromatische Ether, wie Diphenylether, 3) Kohlenwasserstoffe, wie Benzol, Toluol, Xylol, Pseudocumol, Naphthalin, Biphenyl, Tetralin, Dekalin, 4) Ketone wie Aceton, Diethylketon, Methylethylketon oder Methylisobutylketon, 5) Ester, wie Cyclohexylpropionat oder Trimethylenglykoldiacetat. Aber auch 6) Säureamide, wie Dimethylformamid oder N-Methylpyrrolidon, sowie Phenole, Wasser oder das Reaktionsprodukt I selbst sind geeignet.

Bevorzugte Lösungsmittel sind aliphatische Ether, vor allem die Glykol- und Polyglykol-dialkylether, insbesondere die Di-, Tri- oder Tetraethylenglykoldialkylether, deren Alkylgruppen bis zu 6 C-Atomen haben.

Besonders bevorzugt sind die Dimethylether und die Diethylether von Di-, Tri- oder Tetraethylenglykol. Diese Ether haben den Vorteil, daß sie bei atmosphärischem Druck im bevorzugten Temperaturbereich zwischen 180 bis 260°C sieden, was sich für das Verfahren besonders günstig auswirkt, da dann drucklos gearbeitet werden kann und da die Dehydrierung unter Rückflußbedingungen in diesem (bevorzugten) Temperaturbereich besonders rasch und selektiv verläuft.

Es wird in Gegenwart von Substanzen gearbeitet, die als Wasserstoffakzeptoren wirken, d.h. den Wasserstoff sofort bei der Bildung aufnehmen, da dann die als Nebenreaktion ablaufende Hydrierung der Ausgangsverbindung verringert wird.

Als solche Wasserstoffakzeptoren haben sich ungesättigte Verbindungen bewährt wie Isophoren, Styrol, $\alpha$- und $\beta$-Methylstyrol, Stilben, Anthracen, Acenaphthylen, $C_1$—$C_6$-Alkylester von Crotonsäure, Maleinsäure, Fumarsäure, Zimtsäure, sowie Butendiol- und Butindiol-diacetate und -dipropinonate. Auch Nitroverbindungen, wie Nitrobenzol, p-Nitrotoluol oder o-Nitrophenol sind geeignet.

Beim erfindungsgemäßen Verfahren wird der Katalysator im allgemeinen durch heftiges Rühren in der Reaktionslösung suspendiert gehalten.

Wenn das Verfahren mit einem geträgerten Katalysator ausgeführt wird, der im Reaktionsmedium suspendiert ist, so ist im allgemeinen eine Katalysator-Teilchengröße zwischen 0,01 bis 5 mm, vorzugsweise 0,05 bis 1 mm, vorteilhaft. Je nach Natur der Flüssigkeit und des Katalysators enthält die Suspension im allgemeinen 0,1 bis 40 Gewichtsteile geträgerter Katalysator pro 100 Gewichtsteile der Flüssigkeit. Bevorzugte Verhältnisse sind 1 bis 30 Gewichtsteile geträgerten Katalysator pro 100 Gewichtsteile Lösungsmittel.

Das folgende Beispiel dient der Erläuterung der Erfindung.

## Beispiel

### Herstellung von 2,6-Dimethyl-4-hydroxybenzoesäureethylester

Ein Gemisch aus 1700 g Isophoron als Wasserstoffakzeptor, 500 g 3,5-Dimethyl-4-ethoxy-carbonyl-cyclohexen-2-on-1 und 10 g Katalysator, bestehend aus 5 Gew.-% Pd auf Aktivkohle, wurden 5 Stunden lang unter $N_2$-Überlagerung am Rückfluß gekocht. Danach ergab die gaschromatographische Analyse folgende Zusammensetzung (neben 61,0 Gew.-% Isophoron):

| | Gew.-% | Mol |
|---|---|---|
| 1. 3-Methyl-5,5-dimethyl-cyclohexanon | 8,5 | 1,34 |
| 2. 3,5-Dimethyl-4-ethoxycarbonylcyclohexanon | 6,8 | 0,76 |
| 3. 3,5-Dimethyl-4-ethoxycarbonylcyclohexen-2-on-1 | 3,8 | 0,42 |
| 4. 2,6-Dimethyl-4-hydroxybenzoesäureethylester | 20,0 | 2,26 |

Isophoron

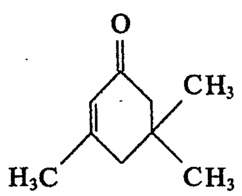

wirkte als Wasserstoffakzeptor, unter Bildung von 3-Methyl-5,5-dimethylcyclohexanon (Produkt 1). Der erfindungsgemäße 2,6-Dimethyl-4-hydroxybenzoesäureethylester (Produkt 4) und 3,5-Dimethyl-4-ethoxycarbonylcyclohexanon (Produkt 2) wurden im Molverhältnis 3 : 1 gebildet.

**Patentansprüche**

1. Verfahren zur Herstellung von 2,6-disubstituierten 4-Hydroxybenzoesäuren bzw. deren Estern der allgemeinen Formel

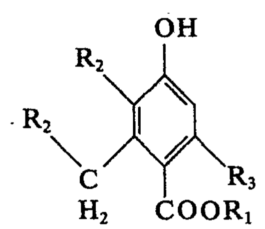

(I)

wobei die Reste $R_1$ und $R_2$ Wasserstoff oder $C_1$—$C_6$-Alkylgruppen, sowie $R_3$ eine $C_1$—$C_6$-Alkylgruppe oder ein Aromat oder Heteroaromat sein können, dadurch gekennzeichnet, daß man Cyclohexenoncarbonsäuren bzw. deren Ester der allgemeinen Formel

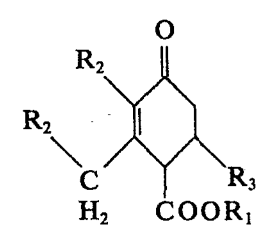

(II)

wobei die Reste $R_1$, $R_2$ und $R_3$ die oben genannte Bedeutung haben, in der Flüssigphase bei Temperaturen zwischen 140 und 350°C in Gegenwart eines Wasserstoffakzeptors an einem Katalysator dehydriert, der ein oder mehrere Edelmetalle der 8. Nebengruppe des Periodensystems enthält.

2. Verfahren nach Anspruch 1, dadurch gekennzeichnet, daß der Dehydrierkatalysator Pd oder Pt enthält.

**Claims**

1. A process for the manufacture of 2,6-disubstituted 4-hydroxybenzoic acids and the esters thereof having the general formula

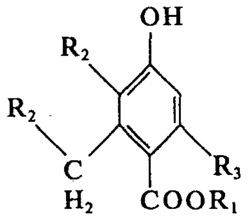

(I)

in which $R_1$ and $R_2$ each are hydrogen or $C_1-C_6$-alkyl, and $R_3$ is $C_1-C_6$-alkyl or an aromatic or heteroaromatic radical, characterized by dehydrogenatiny cyclohexenonecarboxylic acids or the esters thereof having the general formula

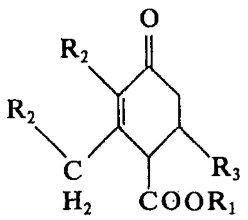

(II)

in which the radicals $R_1$ through $R_3$ are as defined above, in liquid phase at temperatures of from 140 to 350°C in the presence of a catalyst containing one or more noble metals of the 8th subgroup of the Periodic Table and in the presence of a hydrogen acceptor.

2. The process as claimed in Claim 1, wherein the dehydrogenation catalyst contains Pd or Pt.

**Revendications**

1. Procédé de préparation d'acides hydroxy-4-benzoïques disubstitués en 2,6 et de leurs esters, qui répondent à la formule générale I:

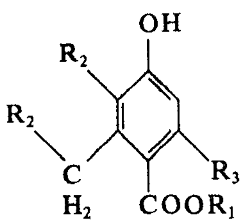

(I)

dans laquelle $R_1$ et $R_2$ représentent chacun l'hydrogène ou un radical alkyle en $C_1-C_6$ et $R_3$ représente un radical alkyle en $C_1-C_6$ ou un radical aromatique ou hétéro-aromatique, procédé caractérisé en ce qu'on déshydrogène des acides cyclohexénone-carboxyliques ou leurs esters répondant à la formule générale II:

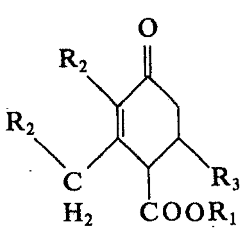

(II)

dans laquelle $R_1$, $R_2$ et $R_3$ ont les significations précédemment données, en phase liquide, à des températures comprises entre 140 et 350°C, en présence d'un accepteur d'hydrogène et d'un catalyseur contenant un ou plusieurs métaux nobles du huitième sous-groupe de la classification périodique.

2. Procédé selon la revendication 1 caractérisé en ce que le catalyseur de déshydrogénation contient du palladium ou du platine.